# FASCICULE DE BREVET EUROPEEN

(11) **EP 1 256 590 B1**
(45) Date de publication et mention de la délivrance du brevet: **01.03.2006**
(21) Numéro de dépôt: 02291853.6
(22) Date de dépôt: 23.07.2002
(51) Int. Cl.: C07K 5/02

(54) **Nouveau procédé de synthèse de dérivés de l'acide (2S, 3aS, 7aS)-1- (S)-alanyl-octahydro-1H-indole-2-carboxylique et application à la synthèse du perindopril**
Verfahren zur Synthese von (2S,3aS,7aS)-1-(S)-Alanyl-octahydro-1H-2-carbonsäurederivaten und Verwendung in der Synthese von Perindopril
Method for synthesis of (2S,3aS,7aS)-1-(S)-alanyl-octahydro-1H-indole-2- carboxylic acid derivatives and use in the synthesis of perindopril

(30) Priorité: 24.07.2001 FR 0109839
(43) Date de publication de la demande: 13.11.2002
(73) Titulaire: Les Laboratoires Servier, 92200 Neuilly sur Seine (FR)
(72) Inventeur: Mezei, Tibor, 1221 Budapest (HU); Porcs-Makkay, Marta, 2013 Pomaz (HU); Simig, Gyula, 1226 Budapest (HU)

(56) Documents cités:
- EP-A- 0 049 658
- EP-A- 0 308 341
- WO-A-01/56353
- WO-A-01/56972
- WO-A-01/58868
- VINCENT M ET AL: "SYNTHESIS AND ACE INHIBITORY ACTIVITY OF THE STEREOISOMERS OF PERINDOPRIL (S 9490) AND PERINDOPRILATE (S 9780)" DRUG DESIGN AND DISCOVERY, HARWOOD ACADEMIC PUBLISHERS GMBH, XX, vol. 9, no. 1, 1992, pages 11-28, XP000885876 ISSN: 1055-9612

## Description

La présente invention concerne un procédé de synthèse industrielle des composés de formule (I) : 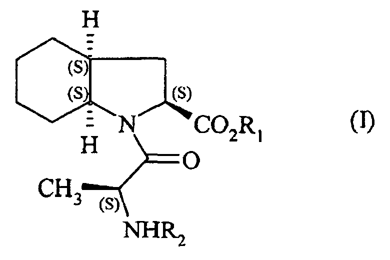 dans laquelle R₁ représente un atome d'hydrogène ou un groupement alkyle (C₁-C₆) linéaire ou ramifié ou benzyle, et R₂ représente un groupement protecteur de la fonction amino,
et leur application à la synthèse industrielle du perindopril de formule (II) : 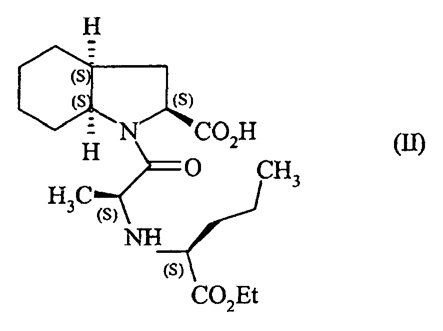 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement, et surtout avec une excellente pureté.
Le brevet EP 0 308 341 décrit la synthèse industrielle du perindopril par hydrogénation catalytique de l'acide (2S)-2,3-dihydroindole 2-carboxylique, suivie du couplage de l'ester benzylique de l'acide (2*S*, 3a*S*, 7a*S*)-octahydroindole 2-carboxylique ainsi obtenu avec l'ester éthylique de la N-[(*S*)-1-carboxybutyl]-(*S*)-alanine, puis de la déprotection du groupement carboxylique de l'hétérocycle par hydrogénation catalytique.
Ce procédé présente l'avantage de conduire au perindopril avec un bon rendement. Cependant, la pureté du perindopril obtenu par ce procédé n'est pas satisfaisante, ce qui nécessite une étape de purification pour parvenir au perindopril avec une qualité permettant son emploi comme principe actif pharmaceutique.
En effet, dans les conditions décrites dans ce brevet, le perindopril obtenu est contaminé dans des proportions importantes par les impuretés de formules (III) et (IV) :

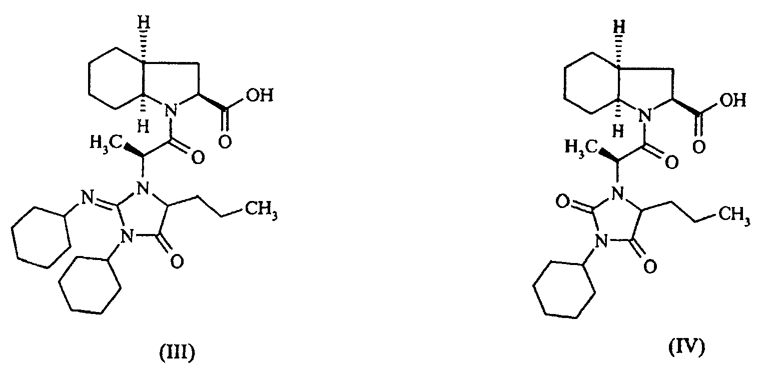

La demanderesse a présentement mis au point un nouveau procédé de synthèse industrielle qui conduit, sans nécessiter de purification laborieuse, au perindopril avec une pureté qui est compatible avec son utilisation comme principe actif pharmaceutique, et qui est notamment totalement exempt des impuretés de formules (III) et (IV).

De plus, ce procédé utilise comme source de chiralité l'alanine, matière première naturelle et donc peu coûteuse.

Plus spécifiquement, la présente invention concerne un procédé de synthèse industrielle du composé de formule (I) : 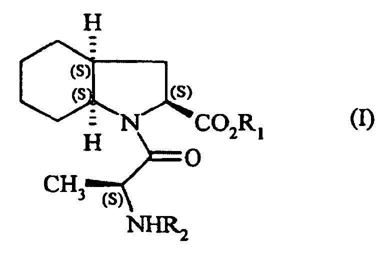 dans laquelle R₁ représente un atome d'hydrogène ou un groupement alkyle (C₁-C₆) linéaire ou ramifié ou benzyle, et R₂ représente un groupement protecteur de la fonction amino,
caractérisé en ce que l'on met en réaction l'ester de formule (V) : 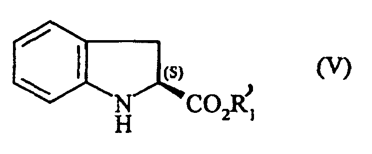 dans laquelle R'₁ représente un groupement alkyle (C₁-C₆) linéaire ou ramifié ou benzyle,
avec le dérivé de l'alanine de formule (VI) : 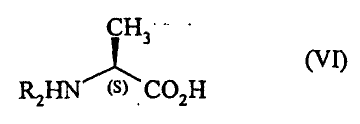 dans laquelle R₂ est tel que défini dans la formule (I),
dans un solvant organique tel que, par exemple, le tétrahydrofurane ou l'acétate d'éthyle, en l'absence de 1-hydroxybenzotriazole ou en présence d'une quantité de 1-hydroxybenzotriazole inférieure à 0,6 mole par mole de composé de formule (V) utilisé, en présence d'une quantité de dicyclohexylcarbodiimide comprise entre 1 et 1,2 mole par mole de composé de formule (V) utilisé, et d'une quantité de triéthylamine comprise entre 1 et 1,2 mole par mole de composé de formule (V) utilisé, à une température comprise entre 20 et 50°C,
pour conduire après isolement puis recristallisation au composé de formule (VII) : 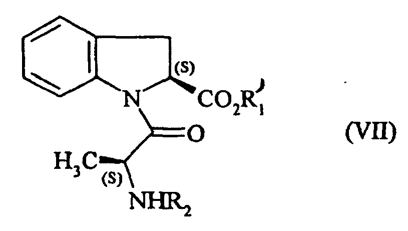 dans laquelle R'₁ et R₂ sont tels que définis précédemment,
que l'on hydrogène en présence d'un catalyseur tel que, par exemple, Pd/C, Rh/C, Pt/C, Ni/C ou PtO₂,
sous une pression d'hydrogène comprise entre 1 et 40 bars, à une température comprise entre 30 et 70°C, pour conduire au composé de formule (I).

Le composé de formule (I) ainsi obtenu est ensuite soumis, le cas échéant, à une réaction de déprotection des fonctions acide et amine, suivie d'une réaction de couplage, soit avec le 2-oxo-pentanoate d'éthyle dans des conditions d'amination réductrice,
soit avec un composé de formule (XII) : 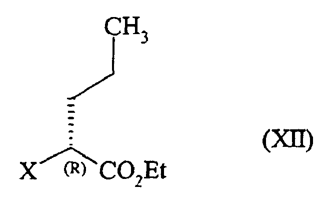 dans laquelle X représente un groupement partant choisi parmi atome d'halogène, ―O―SO₂CH₃ et 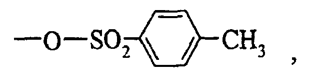 pour conduire au perindopril optiquement pur, que l'on transforme, si on le souhaite, en un sel pharmaceutiquement acceptable tel que le sel de tert-butylamine.

Ce procédé est particulièrement intéressant pour les raisons suivantes :
- Le couplage en milieu alcalin de l'ester benzylique de l'acide (2S, 3aS, 7aS)-octahydroindole 2-carboxylique de formule (VIII) : 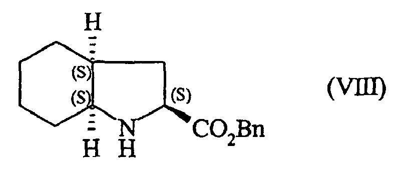 avec le composé de formule (IX) a été décrit dans le brevet EP 0 308 341. 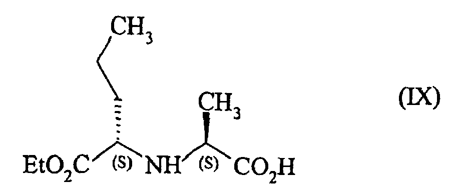
   Cependant, l'ester benzylique du perindopril ainsi obtenu est contaminé par de nombreux produits secondaires.
   En particulier, il contient dans des proportions importantes (5 à 15 %) les impuretés de formules (X) et (XI) résultant de la réaction du produit de couplage avec la dicyclohexylcarbodiimide, impuretés qui après débenzylation conduisent aux impuretés de formules (III) et (IV). 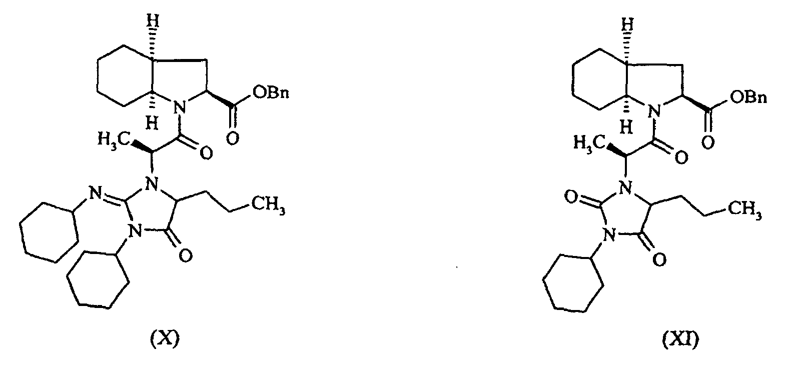
- La Demanderesse a trouvé que la réaction de couplage du composé de formule (V) avec le composé de formule (VI) conduisait à un composé de formule (VII) totalement exempt des impuretés résultant de la réaction du produit de couplage avec la dicyclohexylcarbodiimide.
- Le composé de formule (VII) ainsi obtenu conduit au perindopril avec une pureté bien meilleure, et qui est notamment totalement exempt des impuretés de formules (III) et (IV).
- De plus, la Demanderesse a trouvé que le composé intermédiaire de formule (VU) pouvait être obtenu sous une forme cristalline aisément purifiable.
   Sa transformation selon le procédé de l'invention conduit ainsi au composé de formule (I) avec une pureté excellente.

Les exemples ci-dessous illustrent l'invention, mais ne la limitent en aucune façon.

### Exemple 1: (2S)-1-{(2S)-2-[(Tert-butyloxycarbonyl)-amino]-propionyl}-2,3-dihydro-1H-indole-2-carboxylate de méthyle :

Dans un réacteur sous agitation sont introduits 2,13 kg de (2*S*)-2,3-dihydroindole 2-carboxylate de méthyle, 1 kg de triéthylamine, 30 l de tétrahydrofurane puis, après 10 mn d'agitation à température ambiante, 1,9 kg de N-[tert-butyloxycarbonyl]-(*S*)-alanine, et 2 kg de dicylohexylcarbodiimide. Le mélange hétérogène est ensuite agité à température ambiante pendant 6 heures, puis il est refroidi à 0°C et filtré.
Le filtrat est ensuite lavé, puis recristallisé dans un mélange hexane/acétate d'éthyle 10/1 pour conduire au produit attendu avec un rendement de 81% et une pureté chimique de 98%.
*Point de fusion : 130-131°C*
*Microanalyse élémentaire :*

| | *C%* | *H%* | *N%* |
|---|---|---|---|
| *calculé* | *62,05* | *6,94* | *8,04* |
| *trouvé* | *61,80* | *6,94* | *8,00* |

### Exemple 2 : (2S, 3aS, 7aS)-1-{(2S)-2-[(Tert-butyloxycarbonyl)-amino]-propionyl}-octahydro-1H-indole-2-carboxylate de méthyle / méthode 1 :

Le résidu obtenu dans l'exemple 1 (1 kg) est mis en solution dans le méthanol et transféré dans un hydrogénateur, puis 0,10 kg de rhodium sur charbon à 5% sont ajoutés.
Le mélange est ensuite hydrogéné sous une pression de 30 bars, à une température de 60°C, jusqu'à absorption de la quantité théorique d'hydrogène.
Après filtration du catalyseur, le solvant est évaporé pour conduire au produit attendu avec un rendement de 90% et une pureté chimique de 98%.

### Exemple 3 : (2S, 3aS, 7aS)-1-{(2S)-1-{(Tert-butyloxycarbonyl)-amino]-propionyl}-octahydro-1H-indole-2-carboxylate de méthyle / méthode 2 :

Le résidu obtenu dans l'exemple 1 (1 kg) est mis en solution dans l'acide acétique et transféré dans un hydrogénateur, puis 0,10 kg de dioxyde de platine sont ajoutés.

Le mélange est ensuite hydrogéné sous une pression de 10 bars, à une température de 40°C, jusqu'à absorption de la quantité théorique d'hydrogène.
Après filtration du catalyseur, le solvant est évaporé pour conduire au produit attendu avec un rendement de 90% et une pureté chimique de 98%.

## Revendications

1. Procédé de synthèse industrielle du composé de formule (I) 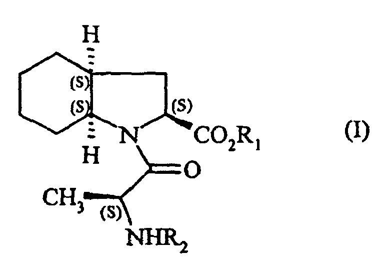 dans laquelle R₁ représente un atome d'hydrogène ou un groupement alkyle (C₁-C₆) linéaire ou ramifié ou benzyle, et R₂ représente un groupement protecteur de la fonction amino,
**caractérisé en ce que** l'on met en réaction l'ester de formule (V) : 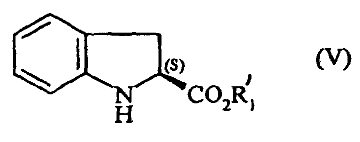 dans laquelle R'₁ représente un groupement alkyle (C₁-C₆) linéaire ou ramifié ou benzyle,
avec le dérivé de l'alanine de formule (VI) : 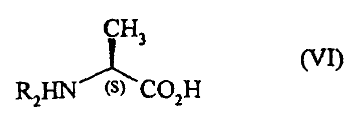 dans laquelle R₂ est tel que défini dans la formule (I),
dans un solvant organique tel que, par exemple, le tétrahydrofurane ou l'acétate d'éthyle, en l'absence de 1-hydroxybenzotriazole ou en présence d'une quantité de 1-hydroxybenzotriazole inférieure à 0,6 mole par mole de composé de formule (V) utilisé, en présence d'une quantité de dicyclohexylcarbodiimide comprise entre 1 et 1,2 mole par mole de composé de formule (V) utilisé, et d'une quantité de triéthylamine comprise entre 1 et 1,2 mole par mole de composé de formule (V) utilisé, à une température comprise entre 20 et 50°C,
pour conduire après isolement puis recristallisation au composé de formule (VII) : 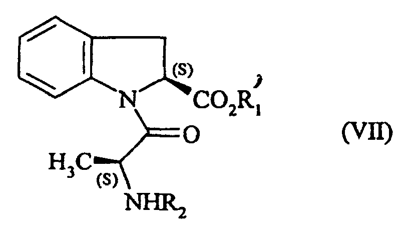 dans laquelle R'₁ et R₂ sont tels que définis précédemment,
que l'on hydrogène en présence d'un catalyseur tel que, par exemple, Pd/C, Rh/C, Pt/C, Ni/C ou PtO₂,
sous une pression d'hydrogène comprise entre 1 et 40 bars, à une température comprise entre 30 et 70°C, pour conduire au composé de formule (I).

2. Procédé de synthèse du composé de formule (I) selon la revendication 1 **caractérisé en ce que** le catalyseur utilisé dans l'étape d'hydrogénation est le rhodium sur charbon.

3. Procédé de synthèse du composé de formule (I) selon la revendication 1, **caractérisé en ce que** le catalyseur utilisé dans l'étape d'hydrogénation est le dioxyde de platine.

4. Procédé de synthèse du composé de formule (I) selon l'une quelconque des revendications 1 à 3, **caractérisé en ce que** R₁ représente le groupement méthyle.

5. Procédé de synthèse du perindopril ou de ses sels pharmaceutiquement acceptables à partir du composé de formule (I), **caractérisé en ce que** le composé de formule (I) est obtenu par le procédé de synthèse selon l'une quelconque des revendications 1 à 4.

6. Procédé de synthèse selon la revendication 5, **caractérisé en ce que** le perindopril obtenu est exempt des impuretés de formules (III) et (IV).

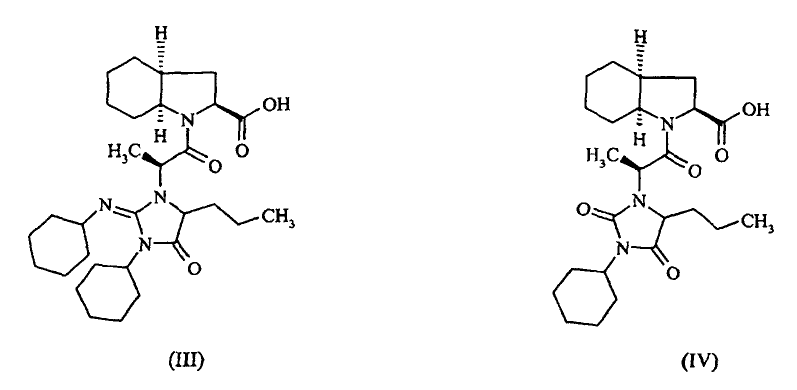

## Patentansprüche

1. Verfahren zur technischen Synthese der Verbindung der Formel (I) 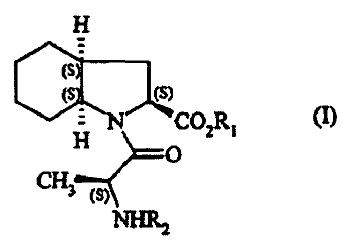 in der R₁ ein Wasserstoffatom oder eine geradkettige oder verzweigte (C₁-C₆)-Alkylgruppe oder Benzylgruppe und R₂ eine Schutzgruppe für die Aminofunktion bedeuten,
**dadurch gekennzeichnet, daß** man den Ester der Formel (V): 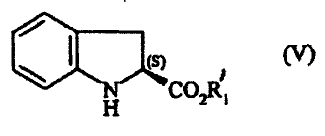 in der R'₁ eine geradkettige oder verzweigte (C₁-C₆)-Alkylgruppe oder Benzylgruppe bedeutet,
mit dem Alaninderivat der Formel (VI): 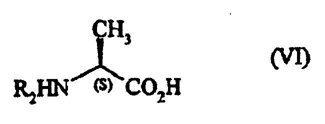 in der R₂ die bezüglich der Formel (I) angegebenen Bedeutungen besitzt,
in einem organischen Lösungsmittel, wie beispielsweise Tetrahydrofuran oder Ethylacetat, in Abwesenheit von 1-Hydroxybenzotriazol oder in Gegenwart von 1-Hydroxybenzotriazol in einer Menge von weniger als 0,6 Mol pro Mol der verwendeten Verbindung der Formel (V), in Gegenwart von Dicyclohexylcarbodiimid in einer Menge zwischen 1 und 1,2 Mol pro Mol der verwendeten Verbindung der Formel (V) und einer Menge von Triethylamin zwischen 1 und 1,2 Mol pro Mol der verwendeten Verbindung der Formel (V),
bei einer Temperatur zwischen 20 und 50 °C umsetzt,
so daß man nach der Umkristallisation die Verbindung derFormel (VII) erhält: 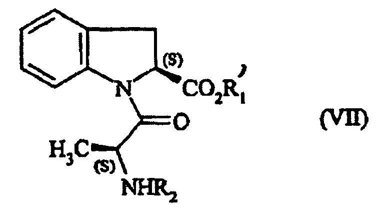 in der R'₁ und R₂ die oben angegebenen Bedeutungen besitzen,
welche man in Gegenwart eines Katalysators, wie beispielsweise Pd/C, Rh/C, Pt/C, Ni/C oder PtO₂,
bei einem Wasserstoffdruck zwischen 1 und 40 bar bei einer Temperatur zwischen 30 und 70 °C hydriert zur Bildung der Verbindung der Formel (I).

2. Verfahren zur Synthese der Verbindung der Formel (I) nach Anspruch 1, **dadurch gekennzeichnet, daß** der in der Hydrierstufe verwendete Katalysator Rhodium-auf-Kohlenstoff ist.

3. Verfahren zur Synthese der Verbindung der Formel (I) nach Anspruch 1, **dadurch gekennzeichnet, daß** der in der Hydrierstufe verwendete Katalysator Platindioxid ist.

4. Verfahren zur Synthese der Verbindung der Formel (I) nach einem der Ansprüche 1 bis 3, **dadurch gekennzeichnet, daß** R₁ die Methylgruppe bedeutet.

5. Verfahren zur Synthese von Perindopril oder von seinen pharmazeutisch annehmbaren Salzen ausgehend von der Verbindung der Formel (I), **dadurch gekennzeichnet, daß** die Verbindung der Formel (I) nach dem Verfahren gemäß einem der Ansprüche 1 bis 4 hergestellt wird.

6. Syntheseverfahren nach Anspruch 5, **dadurch gekennzeichnet, daß** das erhaltene Perindopril frei ist von den Verunreinigungen der Formeln (III) und (IV)

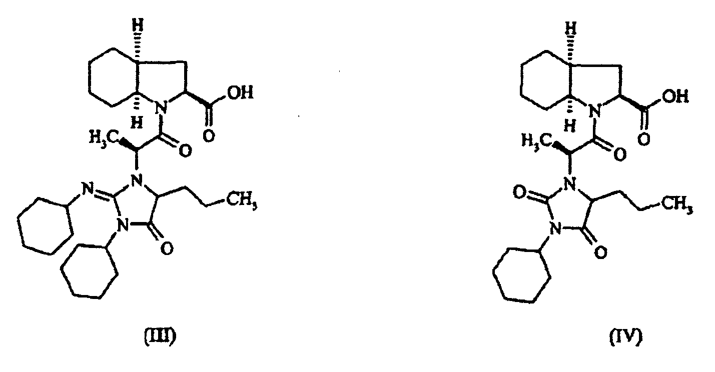

## Claims

1. Process for the industrial synthesis of the compound of formula (I) 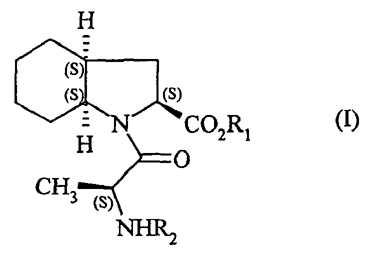 wherein R₁ represents a hydrogen atom, a linear or branched (C₁-C₆)alkyl group or a benzyl group, and R₂ represents a group that protects the amino function,
**characterised in that** the ester of formula (V) : 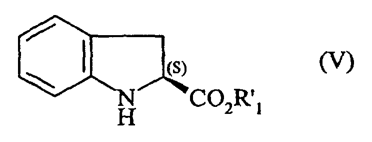 wherein R'₁ represents a linear or branched (C₁-C₆)alkyl group or a benzyl group,
is reacted with the alanine compound of formula (VI) : 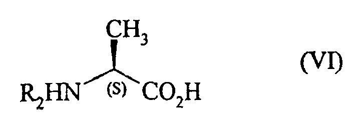 wherein R₂ is as defined for formula (I),
in an organic solvent, such as, for example, tetrahydrofuran or ethyl acetate,
in the absence of 1-hydroxybenzotriazole or in the presence of an amount of less than 0.6 mol of 1-hydroxybenzotriazole per mol of compound of formula (V) employed, in the presence of an amount of from 1 to 1.2 mol of dicyclohexylcarbodiimide per mol of compound of formula (V) employed, and in the presence of an amount of from 1 to 1.2 mol of triethylamine per mol of compound of formula (V) employed,
at a temperature of from 20 to 50°C,
to yield, after isolation and then recrystallisation, the compound of formula (VII) : 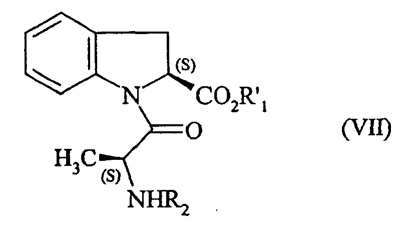 wherein R'₁ and R₂ are as defined hereinbefore,
which is hydrogenated in the presence of a catalyst, such as, for example, Pd/C, Rh/C, Pt/C, Ni/C or PtO₂,
under a hydrogen pressure of from 1 to 40 bars, at a temperature of from 30 to 70°C, to yield the compound of formula (I).

2. Process for the synthesis of the compound of formula (I) according to claim 1, **characterised in that** the catalyst employed in the hydrogenation step is rhodium-on-carbon.

3. Process for the synthesis of the compound of formula (I) according to claim 1, **characterised in that** the catalyst employed in the hydrogenation step is platinum dioxide.

4. Process for the synthesis of the compound of formula (I) according to any one of claims 1 to 3, **characterised in that** R₁ represents a methyl group.

5. Process for the synthesis of perindopril or pharmaceutically acceptable salts thereof starting from a compound of formula (I), **characterised in that** the compound of formula (I) is obtained by the synthesis process according to any one of claims 1 to 4.

6. Synthesis process according to claim 5, **characterised in that** the perindopril obtained is free of the impurities of formulae (III) and (IV).
